# EUROPEAN PATENT SPECIFICATION

(11) **EP 1 858 336 B1**
(45) Date of publication and mention of the grant of the patent: **08.11.2017**
(21) Application number: 06724958.1
(22) Date of filing: 07.03.2006
(51) Int. Cl.: A21D 8/04, C12P 39/00, A61K 35/74, A21D 10/00, A21D 13/04, A21D 2/36, A21D 13/06

(54) **MIXTURE OF AT LEAST 8 SPECIES OF LACTIC ACID BACTERIA AND/OR BIFIDOBACTERIA IN THE MANUFACTURE OF SOURDOUGH**
MISCHUNG VON MINDESTENS 8 MILCHSÄUREBAKTERIEN- UND/ODER BIFIDOBAKTERIEN-ARTEN BEI DER HERSTELLUNG VON SAUERTEIG
MELANGE D'AU MOINS HUIT ESPECES DE BACTERIES DE L'ACIDE LACTIQUE ET/OU DE BIFIDOBACTERIES DANS LA FABRICATION DU LEVAIN

(30) Priority: 16.03.2005 WO PCT/IT2005/000144
(43) Date of publication of application: 28.11.2007
(73) Proprietor: ACTIAL FARMACEUTICA S.r.l., 00144 Roma (IT); VSL Pharmaceuticals, Inc., Towson, MD 21204 (US)
(72) Inventor: DE SIMONE, Claudio, I-00040 Ardea (IT); PIROVANO, Franco, I-20050 Sulbiate (IT)
(74) Representative: Spadaro, Marco
(86) International application number: PCT/EP2006/060505
(87) International publication number: WO 2006/097415

(56) References cited:
- EP-A- 0 856 259
- WO-A-00/54788
- WO-A-02/065842
- WO-A-03/010297
- WO-A-03/071883
- UA-A- 69 137
- US-A- 5 185 165
- US-A1- 2003 215 467
- US-A1- 2004 265 291
- OTTOGALLI G ET AL: "ITALIAN BAKERY PRODUCTS OBTAINED WITH SOUR DOUGH: CHARACTERIZATION OF THE TYPICAL MICROFLORA" ADVANCED IN FOOD SCIENCES, TECHNISCHE UNIVERSITAET MUENCHEN, MUENCHEN, DE, vol. 18, no. 5/6, 1996, pages 131-144, XP008048867 ISSN: 1431-7737
- LUC DE VUYST AND PATRICIA NEYSENS: "The sourdough microflora: biodiversity and metabolic interactions" TRENDS IN FOOD SCIENCE AND TECHNOLOGY, vol. 16, January 2005 (2005-01), pages 43-56, XP002391881 GBELSEVIER SCIENCE PUBLISHERS
- DATABASE WPI Section Ch, Week 200343 Derwent Publications Ltd., London, GB; Class B04, AN 2003-455529 XP002391996 & RU 2 196 173 C2 (MOSC EPIDEMIOLOGY & MICROBIOLOGY INST) 10 January 2003 (2003-01-10)
- GOBBETTI, M. ET AL.: "Biochemistry and physiology of sourdough lactic acid bacteria" TRENDS IN FOOD SCIENCE AND TECHNOLOGY, vol. 16, January 2005 (2005-01), pages 57-69, XP002391882 GBELSEVIER SCIENCE PUBLISHERS
- HSUEH, W., ET AL.: "Neonatal necrotising enterocolitis: clinical considerations and pathogenic concepts" PEDIATRIC AND DEVELOPMENTAL PATHOLOGY, vol. 6, 2002, pages 6-23, XP002391883 USSPRINGER VERLAG,
- HENRY M C W ET AL: "Current issues in the management of necrotizing enterocolitis" SEMINARS IN PERINATOLOGY, W.B. SAUNDERS, GB, vol. 28, no. 3, June 2004 (2004-06), pages 221-233, XP004666165 ISSN: 0146-0005
- DI CAGNO, ET AL.: "Sourdough bread made from wheat and nontoxic flours and started with selected lactobacilli", APPLIED AND ENVIRONMENTAL MICROBIOLOGY, vol. 70, no. 2, 2004, pages 1088-1096, USWASHINGTON,DC ISSN: 0099-2240
- MIMURA, ET AL.: "Once daily high dose probiotic therapy (VSL#3) for maintaining remission in recurrent or refractory pouchitis.", GUT., vol. 53, 2004, pages 108-114, GBBRITISH MEDICAL ASSOCIATION, LONDON, ISSN: 0017-5749

## Description

The present invention relates to the manufacture of baked goods and, more in general, starchy food. It provides baked goods and other food-which are more digestible, they are gluten-free or have a reduced and markedly hydrolyzed gluten content and are particularly suitable non-therapeutic use by subjects affected by celiac disease.

### Background of the invention

Cereals are important components of the daily diet. Nevertheless wheat flour gluten, and in particular the gliadin fraction, are responsible for human intolerance. The celiac disease, also known as Celiac Sprue (CS) or gluten-sensitive enteropathy, is one of the diffuse food intolerance, occurring in 1 out of every 130 to 300 persons of the European and U.S. populations. In South America, North Africa and Asia, is generally underestimated (Fasano and Catassi; 2001, Gastroenterology, 120:636-651)*.* The epidemiological distribution of CS is efficiently conceptualized by the iceberg model introduced by Logan in 1992 (Logan; 1992, Dyn. Nutr. Res. 2:14-24) where the prevalence of the disease is influenced by the frequency of the predisposing genotypes in the population. Total lifelong avoidance of gluten ingestion remains the cornerstone of CS treatment. The International Food Authority has now redefined the term gluten-free as zero tolerance for gluten, while the Codex Alimentarius permits a concentration of 200 ppm of gluten per food. Efforts to reduce the human intolerance to cereals are of a great medical, nutritional and economic interest. This is particularly true in the current context where the bakery industries are using very fast technological processes which may have an influence in the expanding epidemiology of CS.

Therefore, the need of more digestible and tolerated bread and baked products is really felt.

CS is an autoimmune disease of the small intestinal mucosa in genetically susceptible persons. Upon ingestion of gluten, these patients suffer from a self-perpetuating mucosal inflammation characterized by progressive loss of absorptive villi and hyperplasia of the crypts (Silano and De Vincenzi, 1999; Nahrung, 43:175-184)*.* During endoluminal proteolytic digestion, for instance gliadins of wheat release a family of Pro- and Glu-rich oligopeptides that are responsible for the T-cell mediated immune response and/or, more in general, for the inflammatory state which characterizes the initial stage of CS *(Silano and De Vincenzi; 1999).* The literature reports the identification of the following oligopeptides: fragment 31-43 of the A-gliadin (*Silano and De Vincenzi; 1999*), fragment 62-75 of the α2-gliadin (Auricchio, S., et al.; 1996, Scand. J. Gastroenterol., 31:247-253*;* Picarelli, A., et al.; 1999, Scand. J. Gastroenterol., 34:1099-1102), the epitope 33-mer, which corresponds to the fragment 57-89 of the α2-gliadin (Shan, L., et al.; 2002. Science, 297:2275-2279*),* fragment 134-153 of γ-gliadin (Aleanzi, M., et al.; 2001, Clin. Chem., 47: 2023-2028) and the fragment 57-68 of α9-gliadin (Arentz-Hansen, et al.; 2000, J. Exp. Med., 191:603-612). Flours that are not tolerated by CS patients included wheat, rye, barley, kamut, triticale and spelt. Some controversy is still debated for oat.

Multidisciplinary research efforts are carried out in several fields to manage with CS. They concern the engineering of gluten free-grains (Fasano, A., et al.; 2003, Arch. Intern. Med., 163:286-292), search for the CS genes in humans (*Fasano*, *A.*, *et al.; 2003*)*,* use of some protective substances such as mannans and oligomers of N-acetylglucosammine and the use of a bacterial prolyl-endopeptidase from *Flavobacterium meningosepticum* as an oral supplementary therapy (*Shan, et al.; 2002*)*.*

More recently, two articles showed the extensive hydrolysis of the gliadin fractions by selected sourdough lactobacilli such as *Lactobacillus alimentarius* 15M, *L. brevis* 14G, *L. sanfranciscensis* 7A and *L. hilgar*dii 51B (Di Cagno, et al.; 2002, Appl. Environ. Microbiol., 68:623-33) and, especially, the tolerance of 17 CS patients to breads which contained 2 g of gluten, as determined by acute *in vivo* challenges based on the intestinal permeability (Di Cagno, et al.; 2004, Appl. Environ. Microbiol., 70:1088-1096). These results, although encouraging, at least in view of symptomatology, did not prove regression of histopathological damage.

Wei et al. (Wei, J. and Hemmings, G. P.; Medical Hypotheses, (2005), 64, 547-552) establish a genetic relationship between celiac disease and schizophrenia and report the beneficial effect of regression of schizophrenic symptoms in celiac patients treated with gluten-free diet (De Santis, A., et al.; J. Intern. Med. 1997; 242: 421-3)*.*

The use of certain lactic acid bacteria in the manufacture of baked goods is already known.

US 4,140,800, to Kline, discloses a process for making a freeze-dried sourdough bakery starter composition, which uses *Lactobacillus sanfrancisco,* with the aim to provide a product useful in the preparation of French bread. Desirably, the flour is high gluten.

The solution provided by Di Cagno et al., of using sourdough lactic acid bacteria still leave some practical problems unresolved. In particular, (i) selected strains are the results of a very long time consuming research which showed marked differences at strain level within the above species of sourdough lactic acid bacteria; (ii) the same results are obviously non reproducible at bakery plant; and, especially (iii) the selected strains are not commercially available.

Other references disclose the use of lactic acid bacteria and Bifidobacteria in food manufacturing.

EP 0 856 259 relates to a composition for feed use containing a mixture of lyophilized live bacteria comprising at least two species of bacteria selected from Bifidobacteria and at least two species of bacteria selected from Lactobacillus acidophilus, Streptococcus thermophilus, Lactobacillus bulgaricus, Lactobacillus casei, Lactobacillus plantarum and Streptococcus faecium and one or more oligosaccharides. The composition is added to a liquid, creamy or pasty foodstuff, said foodstuff being a milk, a milk-based or milk-derived product, or a product based on or derived from vegetable products, said supplementation being carried out at the moment of use of the foodstuff. The product is not used in bakery.

WO 03/071883 relates to dietetic and/or pharmaceutical compositions for human and/or animal use, and general foodstuffs, based on microbial cultures consisting of autochthonous and allochthonous species with respect to human beings and animals, selected from species of lactic bacteria, propionibacteria, yeasts and/or molds. They have an equilibrating action of the intestinal flora of the host human being or animal, as well as having various beneficial/probiotic effects towards the host organism. There is no indication of a possible use in celiac disease.

US 2004/265291 provides compositions, kits, and methods for providing or restoring beneficial bacteria to a subject. The compositions and kits optionally include food or nutrients to promote growth and proliferation of the bacteria in the subject or an antimicrobial agent to reduce the presence of undesirable or pathogenic microbes in the subject.

WO 02/065842 relates to starter preparations suitable for all types of cereal and the use of the same for producing bread and bakery products based on leaven or using leaven, especially for producing gluten-free bakery products for people with coeliac disease. There is no disclosure of particular mixtures of lactic acid bacteria and Bifidobacteria.

US 5,185,165 discloses a precursor base for use in a bakery dough product comprising an acidic concentrate, at least one type of sugar, yeast, at least one type of flour, non-fat dry milk and at least one type of lactic acid producing bacteria and a process for producing the precursor base are disclosed. The precursor base is useful in a process for producing a precursor slurry (or active ferment concentrate) for use in making a preferment dough mixture for the preparation of the bakery dough product. In addition, processes for preparing the precursor slurry and the preferment dough mixture and an apparatus for producing the preferment dough mixture are disclosed. The reference to lactica acid bacteria is totally generic.

US 2004/110270 describes a bacterial composition having immunomodulation properties comprising at least one strain selected from the group consisting of *Lactobacillus acidophilus* PTA-4797, *Lactobacillus plantarum* PTA-4799, *Lactobacillus salivarius* PTA-4800, *Lactobacillus paracasei* PTA-4798, *Bifidobacterium bifidum* PTA-4801 and *Bifidobacterium lactis* PTA-4802.

EP 1 258 526 discloses the production of a starter for making wheat predough and wheat sourdough by partially fermenting a mixture of water and milled wheat product(s) with an inoculum comprising lactobacilli and yeast comprises using an inoculum comprising an adapted mixed flora including at least one yeast strain, at least one homofermentative lactobacillus strain and at least one heterofermentative lactobacillus strain. There are provided strains of *Saccharomyces* sp. DSM 14265, *Lactobacillus pontis* DSM 14269, *Lactobacillus pontis* DSM 14272, *Lactobacillus pontis* DSM 14273, *Lactobacillus pontis* DSM 14274, *Lactobacillus crispatus* DSM 14271, *Lactobacillus plantarum* DSM 14268 and *Lactobacillus sanfranciscensis* DSM 14270; an adapted mixed flora comprising *Saccharomyces sp.* DSM 14265 and at least three of *Lactobacillus pontis* DSM 14269, *Lactobacillus pontis* DSM 14272, *Lactobacillus pontis* DSM 14273, *Lactobacillus pontis* DSM 14274, *Lactobacillus crispatus* DSM 14271, *Lactobacillus plantarum* DSM 14268 and *Lactobacillus sanfranciscensis* DSM 14270. This reference pertains to the general technical filed of bakery products, with no special medical indications.

WO 99/09839 relates to a paste-like composition which is applicable for use as such and as a filling, coating or other component of various food products, and which contains a significant amount of probiotic. The food product is preferably a bakery product, in particular a ryecontaining bread, rusk, biscuit or the like. This reference deals with the generally known aspects of use of probiotics.

The need of a baked product suitable for subjects affected by celiac disease, which can be obtained with an easy, reproducible process of manufacture and with materials reliable, safe and commercially available is still felt in this field.

WO 00/54788, relates to living lactic acid compositions for regenerating the intestinal flora after diarrhoea or treatment with antibiotics. The compositions of the invention contain different strains of bifido bacteria (e. g., B. *longum, B. bifidum, B. adolescentis, B. breve* and *B. infantis*), and of lactobacilli (e.g., *L. acidophilus*, *L. bulgaricus, L. helveticus, L. casel*, *L. plantarum*) and pharmaceutically accepted components and pre-biotics which contributes to the survival of these strains in the stomach and intestine, leading to an effective re-colonization of the intestinal milieu.

Ottogalli et al., Advances in Food Sciences. Technische Universitatet Muenchen Vol. 18, no. 5/6 131-144 (1996), is a review of the different types of bacteria and micro-organisms which characterize the Italian bakery products.

De Vuyst et al., Trend in Food Science and Technology Vol. 16, 43-56 (January 2005), is a review of the bacterial flora in sourdoughs and of the metabolic interactions occurring between different types of sourdough microorganisms.

Database WPI Week 2003 43, relates to a composition comprising six strains of bifidobacteria which are able to rapidly colonize the intestine and can be used to ferment milk and to prepare biologically active supplements.

UA 69137A, relates to a method for treating newborns with ulcerous necrotic enterocolitis by the oral or rectal administration of the multifunctional multiprobiotic Symbiter resistant to antibiotics .. The multiprobiotic Symbiter comprises 14 strains of symbiotic bifidobacteria, lactobacilli, lactococci, and propionic acid bacteria.

Gobbetti et al. Trends in Food Science and Technology Vol. 16, 57-69 (2005), is a review of the biochemistry and physiology of lactic acid bacteria present in sourdoughs. On page 60, *"Proteolysis during sourdough fermentation"*, the Authors describe the proteolytic properties of *Lactobacillus alamentaraus*, *Lactobacillus breves*, *Lactobacillus sanfranciscensis*, and *Lactobacillus hilgardii.* The Authors report that the results obtained using these lactic acid bacteria demonstrated lysis of albumins, globulins and gliadins in the wheat flour used for fermentation. Anyway, this prior art document does not mention bifidobacteria among the bacteria able to lyse wheat flour proteins.

Hsueh et al. Pediatric and Developmental Pathology, Vol. 6, 6-23 (2002) and Henry et al. Seminars in Perinatology, W.B. Saunders GB vol. 28, no. 3, June 2004, 221-233, refer to the beneficial effects of bifidobacteria and lactobacilli administered *per os* in the treatment of necrotizing enterocolitis.

WO2003/010297 and US2003/215467 refer to the prophylaxis and treatment of inflammatory diseases of the gastrointestinal tract by oral administration of selected bifidobacterium strains to patients.

The present invention meets these needs by providing a baked product for subjects affected by celiac disease. However, the baked product finds a general usefulness in human diet because of its higher digestibility.

### Summary of the invention

It has now been found that certain specific mixtures of lactic acid bacteria and Bifidobacteria, of human and milk origin are endowed with the surprising property of being capable of hydrolyzing gliadin and glutenin fractions which are responsible for celiac disease.

These specific mixtures are very useful in the manufacturing of sourdough and provide well-defined bacterial species.

Therefore, it is an object of the present invention the use of specific mixtures of lactic acid bacteria and Bifidobacteria for the manufacture of sourdough and a sourdough comprising said mixture.

Another object of the present invention is represented by a food comprising the above mentioned sourdough, in particular a cereal-based food, more in particular baked goods which are generally more digestible and in particular can be tolerated by CS patients.

Another object of the present invention is a method for manufacturing cereal-based food, in particular baked goods suitable for subjects affected by celiac disease and suitable to prevent contamination of gluten in gluten-free products.

A further object of the present invention is represented by gluten-free cereal-based food, in particular baked goods made of wheat flour when the use of the specific mixtures of lactic acid bacteria and bifidobacteria is implemented under specific conditions with microbial proteolytic enzymes, routinely used in the bakery industries

Another object of the present invention is a food for subjects affected by celiac disease, said food containing the specific mixture of lactic acid bacteria and Bifidobacteria herein disclosed.

Still another object of the present invention is the use of the above mentioned mixtures of lactic acid bacteria and Bifidobacteria for the preparation of a product useful for reducing Platelet Activating Factor (PAF) and other inflammatory cytokines.

These and other objects of the present invention will be now disclosed in detail in the foregoing, also by means of examples and Figures, wherein:

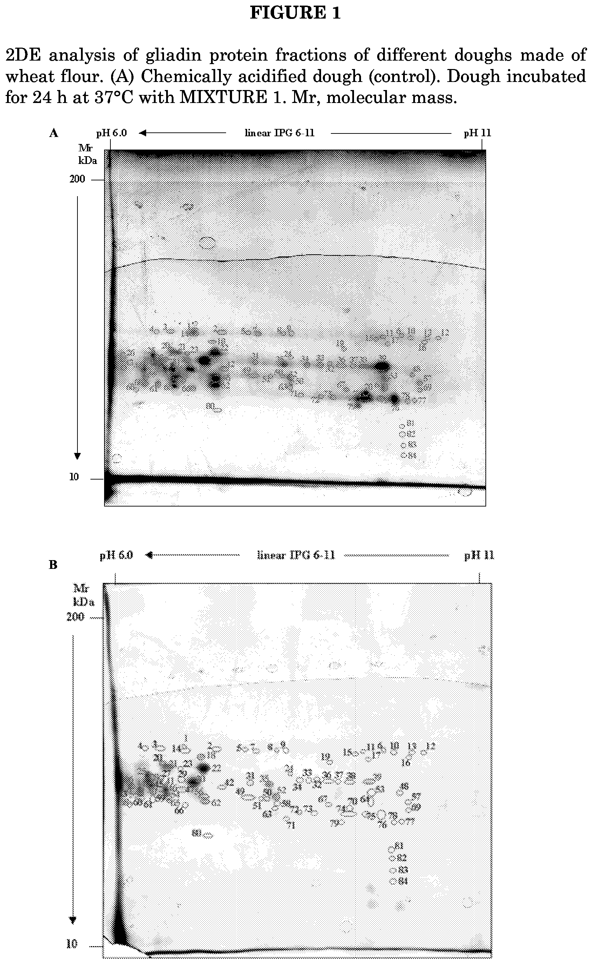

Figure 1 shows 2DE analysis of gliadin protein fractions of different doughs made of wheat flour. (A) Chemically acidified dough (control). Prolamin polypeptides were indicated by numbered red ovals. (B) Dough incubated for 24 h at 37°C with MIXTURE 1 of the Example below. Prolamin polypeptides were indicated by numbered red ovals. Blue numbers refer to polypeptides which were degraded more than 80%. Mr, molecular mass.

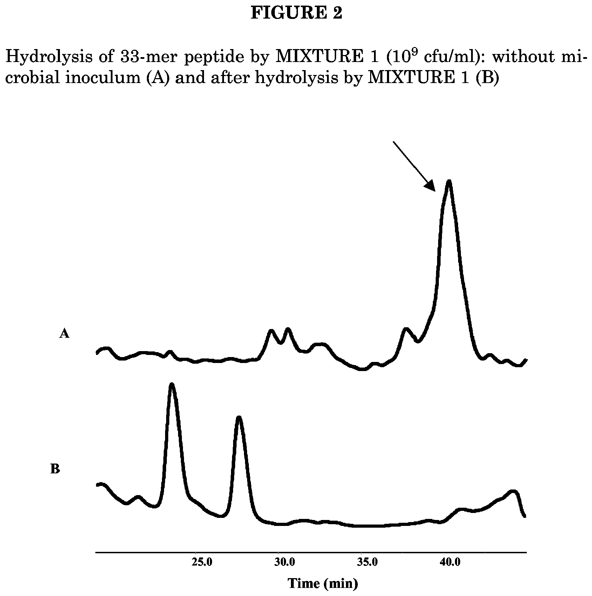

Figure 2 shows hydrolysis of 33-mer peptide by MIXTURE 1 (10⁹ cfu/ml). RP-FPLC at UV 214 nm trace of 200 µM 33-mer after 24 h of incubation at 37°C without microbial inoculum (A) and after 24 h of hydrolysis by MIXTURE 1 at 37°C (B).

### Detailed description of the invention

An object of the present invention, is a sourdough comprising a mixture of at least 8 species of lactic acid bacteria and Bifidobacteria selected from the group consisting of *Lactobacillus acadophalus*, *Lactobacillus caset Lactobacillus delbrueckii* subsp. *bulgaracus*, *Lactobacillus helvetacus*, *Lactobacillus plantarum*, *Bifidobacterium breve, Bifidobacterium infantis*, *Bifidobacterium lactis*, *Bifidobacterium longum, Streptococcus thermophilus* wherein at least one species of lactic acid bacteria and at least one species of bifidobacteria are present, and a microbial proteolytic enzyme, and which has a gluten concentration lower than 200 ppm.

Other species can be used, for example those disclosed in the state of the art and generally available in collections, such as ECACC, ASTM; DSM.

The mixtures according to the present invention are the following:
Streptococcus thermophilus, Bifidobacterium infantis, Bifidobacterium longum, Bifidobacterium breve, Lactobacillus acidophilus, Lactobacillus plantarum Lactobacillus casei, Lactobacillus delbrueckii subsp. bulgaricus.

These mixtures of well known species can be easily prepared by any person having ordinary experience in this field.

Conveniently, these mixtures are commercially available in a lyophilized form.

These formulations are suitable for use as starter in the preparation of sourdough.

The cereal-based food, in particular baked goods obtained according to the present invention are generally more digestible, therefore are more accepted by the general consumer or particularly by people wishing or needing more digestible food.

In a particular embodiment of the invention, the cereal-based food, in particular baked goods can be used for the integration of the diet of people affected by celiac disease, since gluten concentration is reduced to a low value and the amount of gluten which persisted in the dough is markedly hydrolyzed, especially for the peptide sequences which are responsible for CS.

Since the above microbial mixtures are integrated with a sufficient amount of microbial protease, such as for example 200 ppm of microbial protease (typically from *Aspergillus* sp.) under the conditions optimized in the present invention, the fermented sourdough has a concentration of gluten lower than 200 ppm, as determined by using the monoclonal antibody R5. As stated by the Codex Alimentarius such type of product is defined gluten-free and therefore suitable for celiac patients.

Microbial proteases are of common use in bakery, see for example WO 88/03365, EP 0588426, US 6,465,209, GB1,196,946. These proteases are commonly marketed, see for example Enzyme Development Corporation U.S.A. and the present invention can be carried out with any product available on the market and of common use in bakery.

In a preferred embodiment of the present invention, the microbial protease is a fungal protease from *Aspergillus oryzae*; activity of 500,000 HUT/g; pH optimum about 3.0 and activity in the range of pH 3.0 to 6.0; temperature optimum about 50°C and activity in the range 25-60°C; or another protease is an acid stable protease from *Aspergillus niger;* activity of 3,000 SAPU/g; optimum pH 2.0-3.0 and activity in the range of pH 2,0 to 6,0; temperature optimum 50-60°C and activity in the range 30-60°C. These enzymes are available from Bio-Cat Inc., Troy, Virginia, U.S.A. and many other suppliers.

The present invention allows making baked goods with a higher percentage of wheat flour, resulting in products with a more agreeable flavor and better accepted by people affected by celiac disease. The present invention also allows to obtain products directed to general consumers, including healthy people, endowed with better digestibility.

In a wider aspect, the present invention also refers to starchy products comprising the sourdough as disclosed above.

In its widest aspect, the present invention provides a sourdough comprising a mixture of lactic acid bacteria and Bifidobacteria as disclosed above, added with a proteolytic enzyme of microbial origin, useful for the preparation of products for oral administration for improving digestion of gluten and gluten-related substances.

The sourdough comprising the specific mixture of the present invention is the critical aspect of the same.

The sourdough is useful in a process for the preparation of a baked good, in particular bread, but this applies to all leavened and non-leavened products, such as for example biscuits, pastries, cakes, pies, pizza, crackers, breadsticks, snacks and all other products known in the art.

The sourdough according to the present invention is suitable also for preparations for making, also homemade making, cereal-based food, in particular baked goods. In this case, the sourdough may comprise a leavening agent. The present invention also comprises a package for the preparation of the above sourdough, said package comprising a) a gluten-containing flour, b) the above-mentioned mixture containing at least 6 species of lactic acid bacteria and bifidobacteria and c) instructions for use and optionally a leavening agent. The package for a baked good will comprise, other than usual ingredients for the specific product, a leavening preparation comprising the specific mixture of the present invention.

The leavening preparation according to the present invention can be a combination with the specific mixture of lactic acid bacteria and Bifidobacteria or can be provided in the package separately with the lactic acid bacteria and Bifidobacteria mixture and will be mixed with this latter at the moment of use, for example in water to form a leavening suspension. The mixture of lactic acid bacteria can be packaged in a single container alone or in admixture with the proteolytic enzymes (protease) disclosed above.

Starchy products are generally well-known in the field and are part of the common knowledge, also among consumers and homemade cooking. In particular, the present invention is applied to cereal-based products.
Examples of starchy products are all kinds of pasta, noodles, such as fried instant noodles and wet noodles, snack products, tortillas, corn chips, extruded cereals and shredded cereals.

Methods for making pasta are well-known in the art and reference is made just for example to Pasta and Semolina Technology, Edited by R.C. Kill and K. Turnbull, Blackwell Science, 2001 and patents owned by Barilla. Methods for making Asian starchy products are also well-known and just exemplary reference is made to Asian Food, Science and Technology, Edited by Catharina Y.W. Ang, KeShun Liu and Yao-Wen Huang, Technomic Publishing Company, Inc., 1999 and US 20020160093 to Kao Corporation and WO 99/65331, to Societé de Produits Nestlé S.A.

Today, most pasta is manufactured by continuous, high capacity extruders, which operate on the auger extrusion principle in which kneading and extrusion are performed in a single operation. The manufacture of pasta includes dry macaroni, noodle, and spaghetti production.

Pasta products are produced by mixing milled wheat, water, eggs (for egg noodles or egg spaghetti), and sometimes optional ingredients. These ingredients are typically added to a continuous, high capacity auger extruder, which can be equipped with a variety of dies that determine the shape of the pasta. The pasta is then dried and packaged for market.

Pasta products contain milled wheat, water, and occasionally eggs and/or optional ingredients. Pasta manufacturers typically use milled durum wheat (semolina, durum granulars, and durum flour) in pasta production, although farina and flour from common wheat are occasionally used. Most pasta manufacturers prefer semolina, which consists of fine particles of uniform size and produces the highest quality pasta product. The water used in pasta production should be pure, free from offflavors, and suitable for drinking. Also, since pasta is produced below pasteurization temperatures, water should be used of low bacterial count. Eggs (fresh eggs, frozen eggs, dry eggs, egg yolks, or dried egg solids) are added to pasta to make egg noodles or egg spaghetti and to improve the nutritional quality and richness of the pasta. Small amounts of optional ingredients, such as salt, celery, garlic, and bay leafs, may also be added to pasta to enhance flavor. Disodium phosphate may be used to shorten cooking time. Other ingredients, such as gum gluten, glyceryl monostearate, and egg whites, may also be added. All optional ingredients should be clearly labeled on the package.

Durum wheat is milled into semolina, durum granular, or durum flour using roll mills. Semolina milling is unique in that the objective is to prepare granular middlings with a minimum of flour production. After the wheat is milled, it is mixed with water, eggs, and any other optional ingredients.

In the mixing operation, water is added to the milled wheat in a mixing trough to produce dough with a moisture content of approximately 31 percent. Eggs and any optional ingredients may also be added. Most modern pasta presses are equipped with a vacuum chamber to remove air bubbles from the pasta before extruding. If the air is not removed prior to extruding, small bubbles will form in the pasta which diminish the mechanical strength and give the finished product a white, chalky appearance.

After the dough is mixed, it is transferred to the extruder. The extrusion auger not only forces the dough through the die, but it also kneads the dough into a homogeneous mass, controls the rate of production, and influences the overall quality of the finished product. Although construction and dimension of extrusion augers vary by equipment manufacturers, most modern presses have sharpedged augers that have a uniform pitch over their entire length. The auger fits into a grooved extrusion barrel, which helps the dough move forward and reduces friction between the auger and the inside of the barrel. Extrusion barrels are equipped with a water cooling jacket to dissipate the heat generated during the extrusion process. The cooling jacket also helps to maintain a constant extrusion temperature, which should be approximately 51°C (124°F). If the dough is too hot (above 74°C [165°F]), the pasta will be damaged.

Uniform flow rate of the dough through the extruder is also important. Variances in the flow rate of the dough through the die cause the pasta to be extruded at different rates. Products of nonuniform size must be discarded or reprocessed, which adds to the unit cost of the product. The inside surface of the die also influences the product appearance. Until recently, most dies were made of bronze, which was relatively soft and required repair or periodic replacement. Recently, dies have been improved by fitting the extruding surface of the die with Teflon® inserts to extend the life of the dies and improve the quality of the pasta.

Drying is the most difficult and critical step to control in the pasta production process. The objective of drying is to lower the moisture content of the pasta from approximately 31 percent to 12 to 13 percent so that the finished product will be hard, retain its shape, and store without spoiling.

Most pasta drying operations use a preliminary drier immediately after extrusion to prevent the pasta from sticking together. Predrying hardens the outside surface of the pasta while keeping the inside soft and plastic. A final drier is then used to remove most of the moisture from the product.

Drying temperature and relative humidity increments are important factors in drying. Since the outside surface of the pasta dries more rapidly than the inside, moisture gradients develop across the surface to the interior of the pasta. If dried too quickly, the pasta will crack, giving the product a poor appearance and very low mechanical strength. Cracking can occur during the drying process or as long as several weeks after the product has left the drier. If the pasta is dried too slowly, it tends to spoil or become moldy during the drying process. Therefore, it is essential that the drying cycle be tailored to meet the requirements of each type of product. If the drying cycle has been successful, the pasta will be firm but also flexible enough so that it can bend to a considerable degree before breaking.

Packaging keeps the product free from contamination, protects the pasta from damage during shipment and storage, and displays the product favorably. The principal packaging material for noodles is the cellophane bag, which provides moisture-proof protection for the product and is used easily on automatic packaging machines, but is difficult to stack on grocery shelves. Many manufacturers utilize boxes instead of bags to package pasta because boxes are easy to stack, provide good protection for fragile pasta products, and offer the opportunity to print advertising that is easier to read than on bags.

Air emissions may arise from a variety of sources in pasta manufacturing. Particulate matter (PM) emissions result mainly from solids handling and mixing. For pasta manufacturing, PM emissions occur during the wheat milling process, as the raw ingredients are mixed, and possibly during packaging. Emission sources associated with wheat milling include grain receiving, precleaning/handling, cleaning house, milling, and bulk loading. Other information are available in D. E. Walsh and K. A. Gilles, "Pasta Technology", Elements Of Food Technology, N. W. Desrosier, Editor, AVI Publishing Company, Inc., 1977 The present invention is applicable both to the industrial manufacture and the home preparation of pasta, in the latter case, favourably in the preparation of egg pasta.

According to the present invention, in the process of making dough, the mixture of lactic acid bacteria and bifidobacteria herein disclosed are used.

In another embodiment of the present invention, typical Asian, cereal-based food is provided. A preferred example is a kind of noodle known as Ramyun in Korea, Ramien in China and Ramen in Japan.

As in the general carrying out of the present invention, the dough is prepared by adding the mixture of lactic acid bacteria and bifido bacteria and letting the sufficient time for pre-fermentation.

The mixtures of lactic acid bacteria and Bifidobacteria according to the present invention, added with microbial proteases, can also be used in the manufacture of a food, in particular gluten-free grade, for consumption by a subject affected by celiac disease. Examples of this kind of food are pastas, cereals, tacos, tortillas, popcorn. For a reference see Practical Gastroenterology - April 2004, pages 86-104 and the literature cited therein.

Another object of the present invention is a method for the manufacture of a baked good comprising the addition of the above sourdough preparation.

In a preferred embodiment of the present invention, the method comprises the following steps:
a) liquid pre-fermentation of 20-50% of wheat flour by weight (whole mixture 20%-50% of flour and 80%-50% of water, dough yield about 300 with about 10⁹ cells of the mixture of the present invention per g of dough), at about 37°C for at least about 24 h, preferably between about 24 and about 31 hours;
b) after fermentation, mixing the dough with one or more tolerated flour, such as millet flour, to have a final dough yield of about 150 (solid dough) and added of commercial baker's yeast at a concentration of about 1% by weight;
c) incubating the dough at about 37°C for about 2 hours until the leavening is completed;
d) baking at about 250°C for about 20 minutes.

In a second embodiment of the present invention, the method can be modified as follows:
a) liquid fermentation of 20% of wheat flour with the further addition of fungal proteases (200 ppm) at 37°C for 24-31 hours;
b) after fermentation, drying to remove water in order to have a gluten-free wheat flour (< 200 ppm of gluten);
c) use of the wheat flour gluten-free as basic ingredient for the manufacture of cereal-based food, in particular baked goods.

The term "about" in this circumstance means those values around those indicated which are comprised in normal carrying out of the invention and can depend on the instrumental errors of the measuring devices or deviations made by the person skilled in the art around the indicated values, but that do not affect the result obtained by the invention.

The above ranges are intended also as about higher than the lower limit and about lower than the upper limit. Therefore, the liquid pre-fermentation of step a) comprises an amount of wheat flour not lower than about 20% and not higher than 50% by weight for a time of not less than about 24 hours and not more than about 31 hours.

An exemplary list of tolerated flours comprises bean flours, buckwheat, flax, corn (maize), legume flours (garbanzo/chickpea, lentil, pea), millet, Indian Rice Grass, nut flours (almond, hazelnut, pecan), quinoa, potato flour, sweet potato flour, sago, seed flours (sesame), sorghum, soy, tapioca, teff. Millet is one of the preferred tolerated flours.

A very advantageous embodiment of the present invention is to include a prebiotic in the baked good, whether containing or not a tolerated flour.

A prebiotic is a non-digestible fibre-like substance, examples of which are short chain and long chain oligosaccharides, such as fructo-oligosaccharides, soy-oligosaccharides, xylo-oligosaccharides and iso-malto-oligosaccharides.

An even more advantageous embodiment of the invention is the incorporation of the baked good herein disclosed in a baked good such as the one disclosed in EP 1 010 372. In this embodiment, the baked good comprises a non-baked, essentially water-free, fat-based composition comprising live lyophilized lactic bacteria. This fat-based composition comprising live lyophilized lactic bacteria can of course be combined with all the baked products of the present invention.

The cereal-based food, in particular baked goods and the packages for the making cereal-based food, in particular baked goods according to the present invention are suitable for administration to a subject suffering from celiac disease.

As said above, the present invention comprises also general food known under the general name of starchy food, in particular cereal-based food.

The mixture of lactic acid bacteria and bifidobacteria added with the microbial protease, as previously described, is used in the manufacture of starchy food, in particular cereal-based food to obtain the same results and advantages of the above described embodiment of baked goods. So to say, the food obtained according to the present invention is suitable for subjects suffering from celiac disease or for general consumers, also in good health, wishing more digestible food. For example children and ageing people may wish more digestible food.

Therefore, a further object of the present invention is a method for treating a subject suffering from celiac disease comprising the integration of the diet of said subject with a baked good and/or a starchy food as disclosed above. In the foregoing, the baked goods and the starchy food according to the present invention will be comprised in the term "cereal-based food".
In another embodiment of the present invention, the cereal-based food, in particular baked goods can also be used for maintaining gluten tolerance or for inducing gluten tolerance or for decreasing the risk of allergies due to wheat flour albumins and globulins.

In another embodiment of the present invention, the cereal-based food, in particular baked goods can be safety used for celiac patients since the low concentration of gluten (< 200 ppm).

The methods of treatment according to the present invention can also be used in combination with other medical treatments for celiac disease.

As reported above, schizophrenic symptoms are noted in celiac patients and schizophrenic patients show sensitive behavior to gluten. The mixtures according to the present invention are useful for preparing gluten-free dietetic goods.

Therefore, a further object of the present invention is the use of the mixture disclosed above in the preparation of a gluten-free dietetic good useful for the treatment of schizophrenic symptoms. In particular, said symptoms affect a celiac or a non-celiac patient.

Another problem in the art is the use of proline in preparations for enteric diet. In certain subjects, proline is not hydrolyzed and the compounds making the solution for enteric diet are not assimilated. Also allergic responses can occur due to proline. The mixtures of lactic acid bacteria and Bifidobacteria disclosed in the present invention are useful for hydrolyzing proline or proline-enriched peptides, thus making preparations for enteric diet effective and non-allergenic.

Thanks to their properties, the mixtures of lactic acid bacteria and Bifidobacteria disclosed in the present invention are also useful for making gliadin-enriched glutamine solutions hypoallergenic.

In another embodiment of the present invention, it has also been found that the mixtures herein disclosed can be used in the manufacture of a product for lowering the levels of Platelet Activating Factor (PAF) and other inflammatory cytokines for treating gastro-intestinal disease.

PAF is involved in a series of gastro-intestinal diseases, in particular inflammatory disorders. We can mention ischemic bowel necrosis *(*Hsueh W., Gonzalez-Crussi F.; Methods Achiev. Exp. Pathol.; 1988:13; 208-39*),* gastric ulcer (Esplugues Zit., Whittle B.J., Methods Find.; 1989: Suppl. 1, 61-6), hemorrhagic rectocolitis (Chaussade S., Denizot Y, Ann. Gastroenterol. Hepatol. (Paris); 1991, May 27(3): 117-21), necrotizing enterocolitis *(*Ewer AK., Acta Pediatr. Suppl.; 2002, 91(437): 2-5; neonatal: Caplan MS., et al., Semin. Pediatr. Surg.; 2005, Aug 14(3): 154-51*),* inflammatory bowel disease (Nassif A., et al. Dis. Colon Rectum; 1996, Feu.; 39(2):217-23), pouchitis (Rothenberg DA., et al. Ann. Chir.; 1993; 47(10):1043-6).

In view of the above explanation, the product according to the present invention can also be supplemented to subjects, in particular Japanese pople, having a deficit in PAF-hydrolase, who can be affected by a series of inflammatory diseases *(*Karasawa K.; et al.; Prog. Lipid. Res.; 2003 Mar., 42(2): 93-114).
The product can take the form of a food, as disclosed above, or a nutritional supplement, a nutraceutical, a drug.

Nutritional supplement and nutraceutical are well-known terms in the art (Arvanitoyannis IS, et al.; Crit. Rev. Food Sci. Nutr.; 2005,45(5):385-404 and Kalra EK, AAPS PharmSci.; 2003, 5(3); E25) and there is no need of further definition.

The following example further illustrates the invention.

The scope of the present invention is defined strictly by the claims.

### Example 1

### Sourdough fermentation and electrophoresis analyses

The characteristics of the wheat flour used were as follows: moisture, 12.8%; protein (N x 5.70), 10.7% of dry matter (d.m.); fat, 1.8% of d.m.; ash, 0.6% of d.m.; and total soluble carbohydrates, 1.5% of d.m. Eighty grams of wheat flour and 190 ml of tap water (containing a cell concentration of the cell preparations of about 10⁹ cfu per g of dough) were used to produce 270 g of dough (dough yield, 220). Four doughs were manufactured by using the following mixtures of lactic acid bacteria and Bifidobacteria.

### Mixture 1 according to the invention:

*Streptococcus thermophilus,*
*Bifidobacterium infantis*,
*Bifidobacterium longum,*
*Bifidobacterium breve,*
*Lactobacillus acidophilus*,
*Lactobacillus plantarum*
*Lactobacillus casel*,
*Lactobacillus delbrueckii* subsp. *bulgaricus.*

### Mixture 2 not according to the invention

*Streptococcus thermophilus,*
*Bifidobacterium lactis*,
*Bifidobacterium breve,*
*Lactobacillus acidophilus*,
*Lactobacillus plantarum*,
*Lactobacillus casel*,
*Lactobacillus helveticus.*

### Mixture 3

*Lactobacillus acidophilus*,
*Lactobacillus brevis*,
*Streptococcus thermophilus,*
*Bifidobacterium infantis.*

### Mixture 4

*Lactobacillus brevis*,
*Lactobacillus salivarius* spp. *salicinius*
*Lactobacillus plantarum*
Fermentation was carried out at 37°C for 24 h. A dough without bacterial inoculum was chemically acidified to pH 4.0 with a mixture of lactic and acetic acids (molar ratio 4:1) and used as control. After incubation, gliadins were extracted from doughs following the method originally described by Osborne (Osborne, T.B.; 1970, The proteins the wheat kernel. Carnegie Institute of Washington publication 84. Judd and Detweiler, Washington, D.C*.*) and further modified by Weiss et al. *(*Weiss, et al.; 1993, Electrophoresis, 14:805-816).

Aliquots of 10-20 µl (about 10 µg of gliadin) were diluted 1:1 with sample buffer, treated at 100°C for 5 min and analyzed by sodium dodecyl sulfate-polyacrylamide gel electrophoresis (SDS-PAGE) according to the Laemmli procedure (Laemmli; 1970, Nature, 227:680-685); the gels contained 12% of acrylamide and were stained with B10 Bio-Safe Coomassie blue (Bio-Rad Laboratories, Hercules, CA). Two-dimensional gel electrophoresis (2DE) was performed as described by Di Cagno et al. (*Di Cagno; et al*., *2004*). Three gels were analyzed, and spot intensities of chemically acidified dough (siCAD) and sourdough (added of MIXTURE 1) (siSD) were normalized as reported by Bini et al. (Bini, et al.; 1997, Electrophoresis, 18:2832-2841). The hydrolysis factor for individual proteins was expressed as [(siCAD - siSD)/siCAD] x 100. All the hydrolysis factors were calculated based on the average of the spot intensities of the three gels, and standard deviation was calculated. Only hydrolysis factors with statistical significance where *P* value was < 0.05 were reported.

### Hydrolysis of synthetic substrates, Pro-rich polypeptides and RP-FPLC analyses

Preliminarily, the proline specific peptidase activities of Mixture 1 were characterized by using synthetic substrates such as Pro-*p*-NA, Leu-*p*-NA, Ala-*p*-NA, Leu-Leu, Val-Leu, Pro-Gly, Gly-Pro-Ala, Leu-Leu-Leu, Z-Gly-Pro-*p*-NA and NCBZ-Gly-Gly-Leu-*p*-NA (Sigma Chemical Co, St. Louis, Mo). The assay mixture contained 500 µl of 200 mM phosphate buffer, pH 7.5, 150 µl of substrate (0.2-3 mM, final concentration), 8 µl of NaN₃ (0.05% final concentration) and 50 µl of MIXTURE 1 preparation (5 x 10⁹ cfu/ml, final concentration). Fragment 62-75 (P-Q-P-Q-L-P-Y-P-Q-P-Q-S-F-P) of the A-gliadin (*Silano and De Vincenzi; 1999*) and the epitope 33-mer (L-Q-L-Q-P-F-P-Q-P-Q-L-P-Y-P-Q-P-Q-L-P-Y-P-Q-P-Q-L-P-YP-Q-P-Q-P-F) (*Shan et al*., *2002*) were chemically synthesized by Neosystem Laboratoire (Strasbourg, France). The assay mixture for the fragment 62-75 contained 320 µl of 20 mM phosphate buffer, pH 7.0, 150 µl of substrate (450 µM, final concentration), 8 µl of NaN₃ (0.05% final concentration) and 50 µl of MIXTURE 1 preparation (5 x 10⁹ cfu/ml, final concentration). The assay mixture for the epitope 33-mer contained 500 µl of 200 mM phosphate buffer, pH 7.5, 150 µl of substrate (200 µM, final concentration), 8 µl of NaN₃ (0.05% final concentration) and 50 µl of MIXTURE 1 preparation (5 x 10⁹ cfu/ml, final concentration). Both the mixtures were incubated at 37°C under stirred conditions (150 rpm). The enzyme kinetics for the hydrolysis of the 33-mer was calculated by using a Lineweaver-Burk plot (Lineweaver and Burk; 1934, J. American Chem. Soc., 56:658-666)*.*

The enzyme reactions were stopped by addition of 0.05% (vol/vol) (final concentration) trifluoroacetic acid. Peptides were separated from the mixture by RP-FPLC using a Resource II RPC 3ml column and FPLC equipment with a UV detector operating at 214 nm (Amersham Bios-cences, Upssala, Sweden). Elution was at flow rate of 1 ml/min with a gradient (5 to 100%) of acetonitrile in 0.05% trifluoroacetic acid. The concentration of CH₃CN was increased linearly from 5 to 46% between 16 and 62 min and from 46% to 100% between 62 and 72 min.

The same procedure was used to determine the oligopeptides contained in the 70% ethanol-soluble extracts of the fermented doughs.

### Use of fungal proteases in association with lactic acid bacteria and Bifidobacteria

To produce a gluten-free sourdough (< 200 ppm), MIXTURE 1 was used in association with 200 ppm of fungal proteases routinely used in bakery products. During fermentation, the complementary activity of the proteolytic activities from bacteria and fungal sources gave a marked decrease of the of especially gliadin and glutenin fractions. The ethanol-soluble extracts of the fermented sourdough showed a gluten concentration lower than 200 ppm as determined by the use of the monoclonal antibody R5.

### Western blot analysis with R5 monoclonal antibody and RAPD PCR analysis

Fermented dough (37°C for 24 h) with MIXTURE 1 (about 10⁹ cfu per g of dough) was mixed with non protein ingredients and tolerated flour (e.g., millet) to produce Italian biscuits and subjected to baking at 250°C for 15 min. Italian biscuits manufactured without fermentation with MIXTURE 1 were used as control. Biscuits were analyzed by Western blot R5 monoclonal antibody and RAPD PCR at the Centro National de Biotecnologia, Gluten Unit, CNB (28049 Madrid Spain). R5 monoclonal antibody recognizes potential toxic celiac peptides: QQPFP and 33-mer. RAPD PCR was carried based on specific DNA sequences which are related to potential toxic peptides.

### Hydrolysis of wheat flour salt soluble proteins (albumins and globulins)

Albumins and globulins were extracted from wheat flour by the method of Weiss (1993). The assay mixture, containing 0.8 ml of albumins/globulins (about 3 mg/ml) in 50 mM Tris-HCl, pH 7.0, 5 x 10⁹ cfu/ml of MIXTURE 1 and NaN₃ 0.05%. Incubation was at 37°C for 24 h under stirred conditions. A control without microbial cells was included in the test. After incubation, the supernatant was recovered by centrifugation and used for electrophoresis. Proteins from water/salt soluble fraction (albumins and globulins) were analyzed by immunoblotting (Curioni, A., et al., 1999, Clin. Exp. Allergy, 29:407-413) to detect the IgE binding of pooled sera from atopic patients, previously characterized as suffering from gastrointestinal symptoms related to wheat ingestion. By using a semidry blotting, protein bands, separated by SDS-PAGE, were transferred onto nitrocellulose sheets with a Trans-blot Cell (Bio-Rad Laboratories, Milan, Italy) with a transfer buffer containing 48 mM Tris, pH 9.2, 39 mM glycine, 20% methanol and 0.1% SDS, for 5 h at the voltage of 50 V. Blotting bands were visualized by soaking the membranes for a few minutes in Ponceau S (0.1% in 3% trichloroacetic acid) and marked with a pencil, before destaining with water. Membranes were blocked with TBS containing 0.05% Tween 20 (TBS-T) and 5% skim milk powder (M-TBS-T) for 2 h, and incubated overnight with pooled sera from patients, diluted 1:20 in TBS-T. After washing five times with M-TBS-T, blots were incubated for 1 h with monoclonal antihuman IgE peroxidase-conjugate antibody (Sigma Chemical Co), diluted 1:5000 in M-TBS-T (*Curioni*, *et al.; 1999*)*.* After four washes in M-TBS-T and one in TBS, bound IgE were visualized by chemiluminescence using the Supersignal Detection kit (Pierce Biotechnology Inc., Rockford, IL), according to the instructions provided by the manufacturer. The procedure was carried out at room temperature.

Compared to the control, the SDS-PAGE profiles of the gliadin fractions extracted from the doughs fermented with the four cell preparations showed that not all the cell preparations had the same capacity to degrade gliadins. Hydrolysis was very high for MIXTURE 1 of the invention, just slight for MIXTURE 2, while the other cell preparations (MIXTURES 3 and 4) did not cause an appreciable degradation.

The differences among the four cell preparations were confirmed by the RP-FPLC analysis of the 70% ethanol soluble protein fractions which gave an overall view of the oligopeptides with apparent molecular masses lower than those detectable by electrophoresis.

The above results gave a great evidence of the highest performance of the MIXTURE 1 which seemed to have a proteolytic activity more specifically addressed to gliadins.

When the bacterial species which composed MIXTURE 1 were used individually at the same concentration of about 10⁹ cells per g of dough, none of the 8 species gave a marked hydrolysis as shown by the mixture. This was the first evidence of the complementary proteolytic activity between the species of at least 6 strains which are used in MIXTURE 1 in well defined proportion.

Gliadins and related oligopeptides are characterized by a large proportion of proline residues within their sequences (Wieser, 1996, Acta Pediatr. Suppl. 412:3-9)*.* Proline is unique among the 20 amino acids because of its cyclic structure. This specific conformation imposes many restrictions on the structural aspects of peptides and proteins, making them extremely resistant to hydrolysis. To adequately deal with such peptides, a group of specific peptidases is necessary to hydrolyze all the peptide bonds in which a proline residue occurs as potential substrate at the different positions (Cunningham and Connor; 1997, Biochim. Biophys. Acta, 1343:160-186)*.* Preliminarily, the proline specific peptidase activities of MIXTURE 1 were characterized by using synthetic substrates such as Pro-p-NA, Leu-p-NA, Ala-p-NA, Leu-Leu, Val-Leu, Pro-Gly, Gly-Pro-Ala, Leu-Leu-Leu, Z-Gly-Pro-*p*-NA and NCBZ-Gly-Gly-Leu-p-NA which are relatively specific for proline iminopeptidase, aminopeptidase, dipeptidase, prolinase, prolidase, dipeptidyl peptidase, tripeptidase, prolyl-endopeptidase and endopeptidase enzymes, respectively (Table 1).

**Table 1**

| Enzyme activities of MIXTURE 1. | | | |
|---|---|---|---|
| | | | |
| Substrate | Type of enzyme | Substrate concentration (mM) | Unit of activity (U) |
| | | | |
| Pro-*p*-NA | Proline iminopeptidase | 2 | 3.2 ± 0.02 |
| Leu-*p*-NA | Aminopeptidase | 2 | 8.4 ± 0.04 |
| Ala-*p*-Na | Aminopeptidase | 2 | 12.3 ± 0.05 |
| Leu-Leu | Dipeptidase | 2 | 15.51 ± 0.03 |
| Val-Leu | Dipeptidase | 2 | 17.22 ±0.07 |
| Pro-Gly | Prolinase | 3 | 8.0 ± 0.02 |
| Val-Pro | Prolidase | 2 | 3.03 ± 0.02 |
| Gly-Pro-Ala | Dipeptidyl peptidase IV / carboxypeptidase P | 2 | 2.73 ± 0.01 |
| Leu-Leu-Leu | Tripeptidase | 2 | 10.63 ± 0.41 |
| Z-Gly-Pro-*p*-NA | Prolyl-endopeptidase | 2 | 1.3 ± 0.01 |
| NCBZ Gly-Gly-Leu-*p*-NA | Endopeptidase | 2 | 1.9 ± 0.02 |

Each value is the average of three enzyme assays, and standard deviations were calculated. A unit of enzyme activity (U) on *p*-NA substrates was defined as the amount of enzyme which produced an increase in absorbance at 410 of 0.01/min. A unit on polypeptides was the amount of enzyme which liberates 1 micromole of substrates/ min.

All the above enzyme activities were largely distributed in the MIXTURE 1 preparation. Since it is very rare that a unique microbial strain may possess all the previous enzyme activities (*Cunningham and O'Connor; 1997; Kunjii*, *et al.; 1996*, *Antoine Van Leeuwenhoek 70:187-221*; *Di Cagno et al.; 2004*), only a pool of selected bacteria such as those contained in the MIXTURE 1 may have the complete pattern of peptidases needed for hydrolysis of Pro-rich oligopeptides.

The hydrolysis of gliadin oligopeptides by MIXTURE 1 preparation during dough fermentation was further characterized by 2DE analysis. Eighty-four protein spots were identified in the chemically acidified dough used as control (Figure 1A). Seventy-nine of the 84 gliadin oligopeptide spots were markedly degraded after dough fermentation with MIXTURE 1 compared to control (Figure 1B). Table 2 refers to the hydrolysis factors of the spots identified by 2DE. Most of the oligopetides degraded (65 of the 79) had hydrolysis factors higher than 80% and only 8 showed hydrolysis factors lower than 40 %.

**Table 2**

| Properties of alcohol-soluble polypeptides hydrolyzed by MIXTURE 1 after dough incubation at 37°C for 24 h^{a}. | | | |
|---|---|---|---|
| | | | |
| Spot designation^{b} | Estimated pI | Estimated molecular mass (kDa) | Hydrolysis factor |
| 1 | 6.84 | 51.0 | 54.0 |
| 2 | 7.15 | 49.8 | 90.5 |
| 3 | 6.55 | 49.5 | 85.0 |
| 4 | 6.38 | 49.0 | 92.0 |
| 5 | 7.52 | 48.9 | 95.4 |
| 6 | 9.40 | 48.7 | 87.0 |
| 7 | 7.64 | 48.5 | 90.6 |
| 8 | 7.98 | 48.4 | 85.0 |
| 9 | 8.05 | 48.3 | 90.8 |
| 10 | 9.52 | 48.1 | 96.2 |
| 11 | 9.15 | 48.0 | 91.5 |
| 12 | 9.87 | 47.9 | 90.0 |
| 13 | 9.70 | 47.8 | 97.7 |
| 14 | 6.83 | 47.7 | 85.0 |
| 15 | 9.10 | 47.6 | 92.5 |
| 16 | 9.69 | 47.0 | 90.8 |
| 17 | 9.25 | 46.3 | 90.8 |
| 18 | 7.08 | 46.0 | 52.5 |
| 19 | 8.70 | 44.5 | 93.2 |
| 20 | 6.54 | 44.0 | 95.6 |
| 21 | 6.63 | 43.2 | 0.0 |
| 22 | 7.10 | 43.0 | 10.0 |
| 23 | 6.70 | 42.9 | 91.4 |
| 24 | 8.04 | 42.6 | 67.0 |
| 25 | 6.35 | 42.5 | 0.0 |
| 26 | 6.04 | 41.8 | 0.0 |
| 27 | 6.49 | 41.7 | 87.7 |
| 28 | 6.40 | 41.6 | 16.0 |
| 29 | 6.78 | 41.4 | 95.0 |
| 30 | 7.00 | 41.3 | 47.5 |
| 31 | 7.58 | 41.2 | 93.2 |
| 32 | 8.55 | 41.1 | 90.1 |
| 33 | 8.45 | 41.0 | 85.4 |
| 34 | 8.25 | 40.9 | 86.2 |
| 35 | 8.00 | 40.8 | 20.5 |
| 36 | 8.68 | 40.7 | 93.1 |
| 37 | 8.85 | 40.65 | 88.6 |
| 38 | 8.90 | 40.6 | 84.8 |
| 39 | 9.18 | 40.55 | 81.5 |
| 40 | 6.37 | 40.5 | 45.6 |
| 41 | 6.56 | 40.4 | 82.0 |
| 42 | 7.20 | 40.0 | 93.0 |
| 43 | 6.05 | 39.9 | 95.2 |
| 44 | 6.26 | 39.8 | 24.8 |
| 45 | 6.48 | 39.7 | 95.0 |
| 46 | 6.57 | 39.6 | 44.5 |
| 47 | 6.81 | 39.5 | 93.5 |
| 48 | 9.55 | 39.3 | 91.7 |
| 49 | 7.57 | 39.0 | 92.4 |
| 50 | 7.95 | 38.9 | 87.9 |
| 51 | 7.80 | 38.7 | 92.0 |
| 52 | 8.05 | 38.6 | 58.2 |
| 53 | 9.20 | 38.5 | 90.8 |
| 54 | 6.62 | 38.3 | 0.0 |
| 55 | 6.26 | 38.2 | 90.7 |
| 56 | 7.12 | 38.1 | 94.8 |
| 57 | 9.64 | 38.0 | 93.1 |
| 58 | 8.08 | 37.9 | 94.5 |
| 59 | 6.60 | 37.8 | 85.7 |
| 60 | 6.26 | 37.5 | 91.5 |
| 61 | 6.40 | 37.3 | 90.4 |
| 62 | 7.10 | 37.1 | 94.8 |
| 63 | 8.06 | 36.7 | 91.2 |
| 64 | 9.20 | 36.5 | 90.0 |
| 65 | 6.61 | 36.3 | 95.7 |
| 66 | 6.85 | 36.0 | 90.0 |
| 67 | 8.75 | 35.8 | 95.2 |
| 68 | 6.15 | 35.7 | 24.8 |
| 69 | 9.65 | 35.6 | 95.0 |
| 70 | 9.00 | 35.5 | 44.5 |
| 71 | 8.20 | 35.2 | 93.5 |
| 72 | 8.48 | 34.9 | 91.7 |
| 73 | 8.60 | 34.7 | 95.0 |
| 74 | 8.98 | 34.5 | 87.9 |
| 75 | 9.14 | 34.3 | 82.0 |
| 76 | 9.37 | 34.1 | 85.0 |
| 77 | 9.60 | 33.9 | 90.8 |
| 78 | 9.51 | 33.6 | 0.0 |
| 79 | 8.90 | 33.0 | 90.7 |
| 80 | 7.15 | 32.2 | 94.8 |
| 81 | 9.45 | 30.3 | 90.5 |
| 82 | 9.46 | 29.3 | 88.5 |
| 83 | 9.47 | 28.0 | 94.7 |
| 84 | 9.48 | 26.6 | 96.5 |

| | | | |
|---|---|---|---|
| ^{a}Analyses were performed with Image Master software (Pharmacia). Four gels of independent replicates were analyzed. For spot quantification and hydrolysis factor calculation, see Materials and Methods. All of the hydrolysis factors were calculated based on the average of the spot intensities of each of four gels, and standard deviations were calculated. ^{b}Spot designation corresponds to those of the gels in Figure 1A and 1B. | | | |

The above results showed that MIXTURE 1 had the capacity to almost totally hydrolyzed gliadin oligopeptides.

The activity of MIXTURE 1 was further *in vitro* characterized towards some of the oligopeptides reported in the literature as the major responsible for CS: the fragment 62-75 of the A-gliadin (*Silano and De Vincenzi; 1999*) and the epitope 33-mer (*Shan, et al.; 2002*)*.* As shown by the RP-FPLC analysis, the fragment 62-75 of the A-gliadin, at a concentration of 450 µM, was completely hydrolyzed after 6 h of incubation with 5 x 10⁹ cfu/ml cells of MIXTURE 1. The epitope 33-mer, at a concentration of 200 µM, was completely hydrolyzed after 24 h of incubation with the same cell concentration of MIXTURE 1 (Figure 2). The kinetics of hydrolysis of the 33-mer was determined by the Lineweaver-Burk plot showing a Vₘₐₓ of 0.26 µmol per milliliter per min and a Kₘ of 216 µM. As previously reported in the literature, it should be noted that the epitope 33-mer has the following properties: (i) it remains intact despite prolonged exposure to gastric and pancreatic proteases; (ii) it shows a hydrolysis less than 20% over 20 h of incubation with small brush border membrane enzymes; and (iii) it remains intact for a long time (about 24 h) in the small intestine and even at low concentration acts as potential antigen for T-cell proliferation (*Shan, et al. 2002*)*.* The above results showed that MIXTURE 1 contained the complex pool of enzyme activities needed to completely hydrolyze the 33-mer and that these activities are markedly higher than those located at the gastrointestinal level.

Compared to European gliadin references, the Western blot by R5 monoclonal antibody of Italian biscuits had the typical profile of intact gliadin. A major advantage of the R5 monoclonal antibody is its ability to recognize the consensus amino acid sequence QXPW/FP (Osman, et al.; 2001, Eur. J. Gastroenterol. Hepatol., 13: 1189-1193*)* corresponding to multiple immunoreactive epitope repeats, which occur in α-, γ- and ω-gliadins as well as in different wheat varieties (Shewry, et al.; 1992, Cereal's proteins and celiac disease. In: Celiac disease, Marsh M. [ed], Oxford, Blackwell Scientific Publications pp.305-348). Greatest reactivity has been associated with the QQPFP amino acid sequence, but homologous repeats such as LQPFP, QLPYP, QLPTF, QQSFP, QQTFP, PQPPP, QQPYP and PQPFP are also recognized with a weaker reactivity to R5 antibody (*Osman, et al.; 2001*)*.* It is interesting to note that three of these epitopes (LQPFP, QLPYP and PQPFP) are placed in the sequence of the potent inducer of gut-derived human T-cell lines of celiac patients, of the A-gliadin 33-mer peptide (*Shan, et al.; 2002*)*.* The Western blot of the Italian biscuits fermented with the MIXTURE 1 showed an almost degradation of α-, β- and γ-gliadins recognized by R5 monoclonal antibody.

The same results were confirmed by RAPD PCR analysis.

Preliminary experiments for the identification of allergen fractions of wheat albumins and globulins indicated that 100% of the sera tested were positive against albumin and globulin fractions. Responses were found against protein components with apparent molecular masses which ranged from 15 to 70 KDa, with an intense staining for some sera around 15 to 45 KDa. As determined by mono-dimensional SDS-PAGE, the comparison of untreated albumins and globulins with that hydrolyzed by MIXTURE 1 preparation highlighted the hydrolysis of several potential allergens polypeptides.

### Example 2

The sourdough prepared according to Example 1 was used in the manufacture of baked products.

Baked products as disclosed in Examples of US patent 6,884,443 were prepared by using the dough composition according to the present invention instead of the one of the patent. Fermentation was carried out at 37°C for 24 hours as disclosed in Example 1 above and MIXTURE 1 was used.

The products resulted more digestible and suitable for subjects affected by celiac disease.

### Example 3

The sourdough prepared according to Example 1 with MIXTURE 2 was used in the manufacture of pasta.

The pasta resulted more digestible and can be taken by subjects affected by celiac disease.

### Example 4

MIXTURE 1 according to Example 1 was used in the manufacture of noodles according to the teaching of US 2002/0160093.

Examples 1-4 of US 2002/0160093 were repeated, except a packet containing a MIXTURE 1 according to the present invention was added to kansui and flour mixture. After kneading, the mixture was allowed to stand at 37°C for 24 hours. Then noodles were prepared as disclosed in the reference.

The product resulted more digestible and suitable for subjects affected by celiac disease.

### Example 5

MIXTURE 2 according to Example 1 was used in the manufacture of noodles according to the teaching of WO 99/65331.

Examples 1-2 of WO 99/65331 were repeated, except a packet containing a MIXTURE 2 according to the present invention was added to ingredient for the dough. After mixing, the dough was allowed to stand at 37°C for 24 hours. Then noodles were prepared as disclosed in the reference.

The product resulted more digestible and suitable for subjects affected by celiac disease.

### Example 6

MIXTURE 1 according to Example 1 was used in the manufacture of bread derivatives according to the teaching of EP 0 614 609.
Examples 1-5 of EP 0 614 609 were repeated, except a packet containing a MIXTURE 1 according to the present invention was added to dough preparation. After kneading, dough was allowed to stand at 37°C for 24 hours. Then products were prepared as disclosed in the reference.

The product resulted more digestible and suitable for subjects affected by celiac disease.

### Example 7

MIXTURE 2 according to Example 1 was used in the manufacture of spaghetti according to the teaching of EP 1 338 209.

Example of EP 1 338 209 was repeated, except a packet containing a MIXTURE 2 according to the present invention was added to ingredient for the dough. After mixing, the dough was allowed to stand at 37°C for 24 hours. Then noodles were prepared as disclosed in the reference.

The product resulted more digestible and suitable for subjects affected by celiac disease.

### Example 8

### Ramyun

### Composition:

### 1. Noodle

| | |
|---|---|
| Flour: | 83-85% |
| Refined Oil: | 15-18% |
| Salt: | 1% |
| Others: | 0.6-1% |

### 2. Dry Soup Base

Dried Beef Flake, Soy Sauce, Mono Sodium Glutamate, Disodium Glutamate, Flavoring Additive, Glucose, Garlic, Onion, Green Onion, Red Pepper Powder, Other ingredient for flavor

### Manufacturing Process for Ramyun

Flour (sometimes starch, rice flour, barley flour can be used in a different ratio) and water are mixed according to the manufacturer's recommendation. MIXTURE 1 was added to the dough and left to stand at 37°C for 24 hours.

Roll the mixed dough with pressing roller and then put the dough through the machine to make the individual string of noodles. The shape and thickness of the noodle can be modified to the wanted thickness and shape by adjusting the slot size of the shredding machine and also the speed of the convey belt carrying the noodle.

Noodle pass through the steam box of which temperature is more than 100 ° C to induce the pregelatinized starch (a- starch) to make the digestion easier.

After the steaming process, noodle is formed into the certain shape by molding case.

Deep frying process: Depending on the type of Ramyun, the dehydration occurs through deep frying process. The noodle goes through deep frying process at 150°C. Some Ramyun does not go through this deep frying process.

After the deep frying process, the Ramyun goes through cooling process.

According to the present invention, the specific mixture of lactic acid bacteria and Bifidobacteria is suitable for the manufacturing of cereal-based food, in particular baked goods, which may be more tolerated by CS patients. In particular, the following advantages are provided:
(i) marked capacity to degrade gliadin oligopeptides during dough fermentation;
(ii) hydrolysis of 79 of the 84 gliadin oligopeptides identified by 2DE analysis;
(iii) complementary and large enzyme activities towards synthetic peptides which included proline at the different positions;
(iv) capacity to hydrolysis completely oligopeptides (fragment 62-75 of the A-gliadin and epitope 33-mer) which are responsible for CS;
(v) capacity to markedly decrease α-, β- and γ-gliadins which reacted with R5 monoclonal antibody;
(vi) capacity to hydrolyze several allergen polypeptides.
(vii) when the activity of the above bacteria is supplemented with fungal proteases and used towards 20% wheat flour under liquid fermentation, it increases strongly producing gluten-free wheat flour.

## Claims

1. A sourdough comprising a mixture of *Lactobacillus acidophilus, Lactobacillus delbrueckii* subsp. *bulgaricus, Streptococcus thermophilus, Lactobacillus plantarum*, *Lactobacillus casei, Bifidobacterium breve, Bifidobacterium infants*, and *Bifidobacterium* longum, and a microbial proteolytic enzyme of use in bakery industry.

2. The sourdough according to claim 1 in which the proteolytic enzyme is obtained from *Aspergillus sp.*

3. The sourdough according to any one of claims 1-2 further comprising a leavening agent.

4. A food comprising the sourdough of any of claims 1-3.

5. The food according to claim 4, wherein said food is selected from the group consisting of biscuits, pastries, cakes, pies, pizza, crackers, breadsticks, noodles, spaghetti and pasta, ramyun.

6. A package comprising:
a mixture of *Lactobacillus acidophilus, Lactobacillus delbrueckii* subsp. *bulgaricus, Streptococcus thermophilus, Lactobacillus plantarum*, *Lactobacillus casei, Bifidobacterium breve*, *Bifidobacterium infantis*, and *Bifidobacterium longum*, wherein said mixture is in a single container alone or in admixture with a microbial proteolytic enzyme of use in bakery industry.

7. The sourdough according to any one of claims 1-3 or of the food according to any one of claims 4-5 for use as a dietetic and non-allergenic product for enteric diets.

8. The product for use according to claim 7 for further use in inducing or maintaining gluten tolerance in a subject suffering from celiac disease.

9. The product for use according to claim 7 for further use in decreasing the risk of allergies due to wheat flour albumins and globulins.

10. The product for use according to claim 7 for further use in the treatment of schizophrenic symptoms in a celiac patient or a non-celiac patient.

11. The product for use according to claim 7 for further use in reducing Platelet Aggregating Factor (PAF) and/or inflammatory cytokines in gastro-intestinal diseases.

12. The product for use according to claim 11, wherein said disease is an inflammatory disease.

13. The product for use according to claim 11, wherein said disease is selected from the group consisting of ischemic bowel necrosis, gastric ulcer, hemorrhagic rectocolitis, necrotizing enterocolitis, neonatal necrotizing enterocolitis, inflammatory bowel disease and pouchitis.

## Patentansprüche

1. Ein Sauerteig enthaltend eine Mischung aus *Lactobacillus acidophilus*, *Lactobacillus delbrueckii subsp. bulgaricus*, *Streptococcus thermophiles, Lactobacillus plantarum*, *Lactobacillus casei*, *Bifidobacterium breve, Bifidobacterium infantis*, *Bifidobacterium longum* und ein mikrobielles, proteolytisches Enzym aus der Backindustrie.

2. Der Sauerteig nach Anspruch 1, in dem das proteolytische Enzym aus *Aspergillus sp*. gewonnen wurde.

3. Der Sauerteig nach einem der Ansprüche 1 bis 2, weiter enthaltend ein Backtriebmittel.

4. Ein Nahrungsmittel enthaltend einen Sauerteig nach einem der Ansprüche 1 bis 3.

5. Das Nahrungsmittel nach Anspruch 4, wobei das Nahrungsmittel ausgewählt ist aus der Gruppe bestehend aus Keksen, Gebäck, Kuchen, Pasteten, Pizza, Kräcker, Knabberstangen, Nudeln, Spaghetti und Pasta, Ramyun.

6. Eine Packung umfassend:
eine Mischung aus *Lactobacillus acidophilus*, *Lactobacillus delbrueckii subsp. bulgaricus*, *Streptococcus thermophiles, Lactobacillus plantarum*, *Lactobacillus casei, Bifidobacterium breve, Bifidobacterium infantis*, *Bifidobacterium longum*, wobei die Mischung in einem einzelnen Behältnis alleine oder in einer Beimischung mit einem mikrobiellen proteolytischen Enzym aus der Backindustrie ist.

7. Der Sauerteig nach einem der Ansprüche 1 bis 3 oder aus dem Nahrungsmittel nach einem der Ansprüche 4 bis 5 zur Verwendung als diätetisches und nichtallergenes Produkt für enterische Diäten.

8. Das Produkt zur Verwendung nach Anspruch 7 zur weiteren Verwendung zur Induzierung oder dem Erhalt einer Glutentoleranz bei einem an Zöliakie leidenden Individuum.

9. Das Produkt zur Verwendung nach Anspruch 7 zur weiteren Verwendung zur Reduzierung des Risikos von Allergien aufgrund von Weizenmehl-Albuminen und Globulinen.

10. Das Produkt zur Verwendung nach Anspruch 7 zur weiteren Verwendung zur Behandlung von Symptomen der Schizophrenie bei Zöliakiepatienten und Nicht-Zöliakiepatienten.

11. Das Produkt zur Verwendung nach Anspruch 7 zur weiteren Verwendung zur Reduktion des plättchenaktivierenden Faktors (PAF) und /oder inflammatorischer Zytokine bei gastrointestinalen Erkrankungen.

12. Das Produkt zur Verwendung nach Anspruch 11, wobei die gastrointestinale Erkrankung eine Entzündungskrankheit ist.

13. Das Produkt zur Verwendung nach Anspruch 11, wobei die Erkrankung eine Erkrankung ausgewählt aus der Gruppe von ischämischer Darmnekrose, Magengeschwür, hämorrhagische Rektokolitis, nekrotisierende Enterokolitis, neonatale nekrotisierende Enterokolitis, entzündliche Darmerkrankung und Pouchitis ist.

## Revendications

1. Levain comprenant un mélange de *Lactobacillus acidophilus*, *Lactobacillus delbrueckii subsp. bulgaricus*, *Streptococcus thermophilus, Lactobacillus plantarum*, *Lactobacillus casei, Bifidobacterium breve, Bifidobacterium infantis*, et *Bifidobacterium longum,* et une enzyme protéolytique microbienne utilisée dans l'industrie boulangère.

2. Levain selon la revendication 1 dans lequel l'enzyme protéolytique est obtenue à partir d'*Aspergillus sp.*

3. Levain selon l'une quelconque des revendications 1 et 2, comprenant en outre un agent levant.

4. Aliment comprenant le levain selon l'une quelconque des revendications 1 à 3.

5. Aliment selon la revendication 4, dans lequel ledit aliment est choisi dans le groupe constitué de biscuits, pâtisseries, gâteaux, tartes, pizzas, biscuits salés, gressins, nouilles, spaghettis and pâtes et soupes de nouilles.

6. Conditionnement comprenant:
un mélange de *Lactobacillus acidophilus*, *Lactobacillus delbrueckii subsp. bulgaricus*, *Streptococcus thermophilus, Lactobacillus plantarum*, *Lactobacillus casei, Bifidobacterium breve, Bifidobacterium infantis*, et *Bifidobacterium longum,* dans lequel ledit mélange est dans un récipient unique seul ou en mélange avec une enzyme protéolytique microbienne utilisée dans l'industrie boulangère.

7. Levain selon l'une quelconque des revendications 1 à 3 ou aliment selon l'une quelconque des revendications 4 et 5 pour une utilisation en tant que produit diététique et non allergénique pour des régimes alimentaires entériques.

8. Produit pour une utilisation selon la revendication 7 pour une autre utilisation dans l'induction ou le maintien de la tolérance au gluten chez un sujet souffrant de la maladie coeliaque.

9. Produit pour une utilisation selon la revendication 7 pour une autre utilisation dans la diminution du risque d'allergies dues aux albumines et globulines de la farine de blé.

10. Produit pour une utilisation selon la revendication 7 pour une autre utilisation dans le traitement des symptômes schizophréniques chez un patient coeliaque ou un patient non coeliaque.

11. Produit pour une utilisation selon la revendication 7 pour une autre utilisation dans la réduction du facteur d'agrégation plaquettaire (PAF) et/ou de cytokines inflammatoires dans des maladies gastro-intestinales.

12. Produit pour une utilisation selon la revendication 11, dans lequel ladite maladie est une maladie inflammatoire.

13. Produit pour une utilisation selon la revendication 11, dans lequel ladite maladie est choisie dans le groupe constitué de la nécrose ischémique de l'intestin, l'ulcère gastrique, la rectocolite hémorragique, l'entérocolite nécrosante, l'entérocolite nécrosante néonatale, la maladie inflammatoire de l'intestin et la pochite.
